# EUROPEAN PATENT APPLICATION

(11) **EP 1 264 824 A1**
(43) Date of publication of application: **11.12.2002**
(21) Application number: 01113778.3
(22) Date of filing: 06.06.2001
(51) Int. Cl.: C07C 269/04, C07C 271/20

(54) **Preparation of alkyl-N-(3-dimethylamino)alkylcarbamates**

(71) Applicant: Aventis CropScience GmbH, 65929 Frankfurt am Main (DE)
(72) Inventor: Keuchel, Johannes, 64367 Mühltal-Trautheim (DE); Schlegel, Günter, 65835 Liederbach (DE)
(74) Representative: Mérigeault, Shona

(57) **Abstract**

n-Propyl 3-(dimethylamino)n-propylcarbamate by reacting n-propyl chloroformate with 3-(dimethylamino)-n-propylamino in an alcohol.

## Description

### Field of the invention

This invention relates to the preparation of Alkyl-N-(3-dimethylamino)alkylcarbamates.

Alkyl-N-(3-dimethylamino)alkylcarbamates and their salts are used as fungicides (see GB 1 212 708, ZA 68 5172, DE 1 567 169, DE 16 43 040).

n-Propyl-3-(dimethylamino)n-propylcarbamate is a well known fungicidal active ingredient and is normally sold as the hydrochloride salt. This compound is normally prepared by reacting 3-(dimethylamino)n-propylamine with n-propyl chloroformate in inert solvents like toluene, ethers, etc. We have now found a new process which is characterized by carrying out the reaction at varying temperatures in alcohols or mixtures of alcohols with water or/and inert organic solvents.

Thus the invention provides a process for the preparation of n-propyl-3-(dimethylamino)n-propylcarbamate which comprises reacting n-propyl chloroformate in an aliphatic (C₁-C₆)-alcohol. We are able to operate in a wide range of temperatures achieving very high yields.

The fact that this reaction proceeds in such high yields is surprising since the alcohol would be expected to react with the n-propyl chloroformate especially in the presence of an acid acceptor like the 3-(dimethylamino)n-propylamine or n-propyl-3-(dimethylamino)n-propylcarbamate itself, (see for example Houben-Weyl, 4^{th} ed., (1983), E4, page 68). However, in terms of the n-propyl chloroformate a yield of 95% is obtained in this present process and yields based on the amine are even higher (up to 97%). As one would infer, the present invention provides high yields of n-propyl-3-(dimethylamino)n-propylcarbamate compared with processes carried out in inert solvents or water (see DE 16 43 040, DE1 567 169, GB 1 212 708, ZA 68 5172) or other standard carbamate processes (e.g. Houben-Weyl, 4^{th} ed., (1983), 8, p. 138ff and E4, p.149ff).

An additional advantage of the process of the invention is that no additional acid acceptor is required.

Another advantage is that the reaction can be carried out in a wide range of temperatures from -20°C up to the boiling point of the alcohol and even higher, in general up to 200°C. Preferred is about -20°C to about 110°C, especially preferred is about -20°C to about 97°C. These temperatures and therefore reaction speeds are significantly higher than in other processes using only inert solvents and / or water (e.g. Houben-Weyl, 4^{th} ed., (1983), 8, p. 138ff and E4, p.149ff). Often selectivity tends to decrease when using higher temperatures and in the toluene example (see experimental part) the degradation of n-propyl chloroformate increases significantly at temperatures higher than 65°C giving large amounts of carbon dioxide and n-propyl chloride. In other literature examples (see Houben-Weyl, 4^{th} ed. 11/1, p. 985f and E4, p.153f) this degradation is the main reaction even at lower temperatures. It is surprising that in our process the yield in terms of both compounds stays stable even at temperatures above 85°C and that there is very limited degradation of n-propylchloroformate.

Alcohols used according to the invention are (C₁-C₆)-, preferably (C₁-C₄)- aliphatic alcohols. Further preferred are propanols, especially n-propanol, also in recycled form. It is also preferred to use mixtures of one or more alcohols and water, preferably up to 30% by weight, and/or inert organic solvents, like ethers, e.g. THF or methyl t-butyl ether, toluene etc.

The molar ratio of 3-(dimethylamino)n-propylamine to n-propyl chloroformate is in general 1 : 0.75-1.5, preferably 1 : 0.95-1.1.

The process of the present invention is economically and environmentally advantageous due to several other features. Alcohols, like n-propanol are environmentally and technically unproblematic solvents compared to ethers or dichloromethane or other inert solvents. Redistilled alcohol can be used. The reaction can be carried out at a high concentration. The exothermity at the high reaction temperature can be easily controlled. There is almost no gas evolution during reaction and there is no waste water.

The invention is illustrated in the following example.

### Example

3-(dimethylamino)n-propylamine (184.4 g) was added dropwise to recycled n-propanol (443.5 g) at 20-40°C. n-Propyl chloroformate (227.3 g) was added dropwise over 30-40 minutes. The temperature rose quickly and the flask was easily cooled so that the temperature was maintained at 80-85°C. After distilling off the n-propanol (which is later reused) the residue is n-propyl-3-(dimethylamino)n-propylcarbamate hydrochloride in a yield of 97% based on the 3-(dimethylamino)n-propylamine. This is 4% higher than when the reaction is carried out using toluene. The yield was 95% based on the n-propyl chloroformate which is at least 16% higher than when the reaction is carried out with toluene.

### Example for the toluene process:

n-Propyl chloroformate (180 g) was added dropwise to toluene (650 g) at 20-40°C. 3-(dimethylamino)n-propylamine (125 g) was added dropwise over 30-40 minutes. The temperature rose quickly and the flask was cooled so that the temperature was maintained at 55-60°C. The reaction mixture was cooled to 40-45°C and water was added. After phase separation the crude n-propyl-3-(dimethylamino)n-propylcarbamate solution was distilled to give n-propyl-3-(dimethylamino)n-propylcarbamate hydrochloride in a yield of 93% based on 3-(dimethylamino)n-propylamine. The yield was 79% based on the n-propyl chloroformate.

## Claims

1. A process for the preparation of n-propyl 3-(dimethylamino)n-propylcarbamate which comprises reacting n-propyl chloroformate in aliphatic (C₁-C₆) alcohols or mixtures of such alcohols with up to 30% water by weight and/or inert organic solvents.

2. A process according to claim 1 in which n-propanol or recycled n-propanol is the solvent.

3. A process according to claims 1 and/or 2 running the reaction in a temperature range from about -20°C to about 110°C.

4. A process according to any of claims 1 to 3 where both reagents can be used either stoichometric or in a excess up to 50mol%

5. A process according to any of claims 1 to 4 where n-propyl chloroformate is preferably used in an excess of up to 10mol%.
